# EUROPÄISCHE PATENTSCHRIFT

(11) **EP 1 778 331 B1**
(45) Veröffentlichungstag und Bekanntmachung des Hinweises auf die Patenterteilung: **03.03.2010**
(21) Anmeldenummer: 05771415.6
(22) Anmeldetag: 01.08.2005
(51) Int. Cl.: A61M 16/16

(54) **VERDUNSTER SOWIE VERDUNSTUNGSVERFAHREN**
EVAPORATORS AND EVAPORATION METHODS
EVAPORATEURS ET PROCEDES D'EVAPORATION

(30) Priorität: 02.08.2004 DE 102004037698
(43) Veröffentlichungstag der Anmeldung: 02.05.2007
(73) Patentinhaber: Seleon GmbH, 06847 Dessau (DE)
(72) Erfinder: GENGER, Harald, 06846 Dessau (DE); BAECKE, Martin, 06847 Dessau (DE); BAUER, Ulla, 06366 Köthen (DE)
(74) Vertreter: Hellmich, Wolfgang
(86) Internationale Anmeldenummer: PCT/DE2005/001351
(87) Internationale Veröffentlichungsnummer: WO 2006/012877

(56) Entgegenhaltungen:
- DE-A1- 10 016 005
- DE-C1- 3 335 745
- DE-U1- 29 909 611
- US-A- 5 031 612

## Beschreibung

Die Erfindung bezieht sich auf Verdunster der in den Oberbegriffen der Patentansprüche 1 und 19 genannten Art sowie auf Verfahren der in den Oberbegriffen der Patentansprüche 13 und 21 genannten Art. Konkreter bezieht sich die Erfindung auf Luftbefeuchter für Beatmungsgeräte im weiteren Sinne, also auch für den CPAP-, BiLevel- und Antischnarchgerätebereich.

Ein Verdunster sowie ein Verdunstungsverfahren gemäß den Oberbegriffen der unabhängigen Patentansprüche sind beispielsweise aus der DE 199 36 499 A1, DE 200 10 553 U1 oder der DE 101 51 397 C1 bekannt.

Zu den Beatmungsgeräten gehören so genannte CPAP-Geräte, die zur Behandlung von Apnoen (Atemstillstände) während des Schlafs dienen. Hierzu wurde die CPAP (continuous positive airway pressure)-Therapie entwickelt. Ein CPAP-Gerät erzeugt mittels eines Kompressors oder einer Turbine einen positiven Überdruck bis zu etwa 30 mbar und appliziert diesen vorzugsweise über einen Luftbefeuchter, über einen Schlauch und eine Nasenmaske in die Atemwege des Patienten. Dieser Überdruck soll gewährleisten, dass die oberen Atemwege während der gesamten Nacht vollständig geöffnet bleiben und somit keine Apnoen auftreten (DE 198 49 571 A1). Dies wird auch als pneumatische Schienung der Atemwege bezeichnet. Ein in Verbindung mit dem CPAP-Gerät eingesetzter Luftbefeuchter verhindert das Austrocknen der Schleimhäute des Patienten.

Aus der DE 101 05 383 C2 ist ein Antischnarchgerät bekannt, bei dem an Stelle einer Nasen- oder Gesichtsmaske eine Luftbrille verwendet wird. Vorteilhaft an diesem Antischnarchgerät ist der erhöhte Tragekomfort, der sich aus der Vermeidung einer Nasen- oder Gesichtsmaske und der Verwendung dünnerer Schläuche ergibt. Auch sind Luftbrillen Sauerstoffbrillen ähnlich, die bereits kostengünstig in großen Stückzahlen hergestellt werden.

In der DE 199 36 499 A1 ist eine Vorrichtung zur Atemgasbefeuchtung für CPAP-Geräte beschrieben. Die Befeuchtungsvorrichtung umfasst eine aus einem Wannenelement und einem damit gekoppelten Topfteil gebildete Nachfülleinheit, die aus einem Aufstellgehäuse entnommen werden kann. Das Wannenelement und das Topfteil sind miteinander dicht verbunden. In dem Topfteil ist in Verbindung mit einer Trennwand ein Flüssigkeitsvorratsraum gebildet, welcher den überwiegenden Teil des zur Befeuchtung des Atemgases vorgesehenen Wasservorrats enthält. In dem unterhalb des Topfteils angeordneten Wannenelement ist ein separater Befeuchtungsbereich gebildet, in dem lediglich ein kleiner Teil des Wasservorrats enthalten ist. Die Höhe des Wassers im Wannenelement wird über eine Dosiereinrichtung auf einem vorbestimmten Niveau gehalten. Im Zuge der allmählichen Verdunstung des in dem Wannenelement befindlichen Wassers wird sukzessive oder kontinuierlich aus dem Flüssigkeitsvorratsraum Wasser nachgefüllt. Das Atemgas wird über eine Atemgaszutrittsöffnung durch den oberen Bereich des Wannenelements zu einer Atemgasaustrittsöffnung geblasen. Der Bodenbereich des Wannenelements wird durch eine Heizeinrichtung beheizt.

Die DE 200 10 553 U1 beschreibt einen ähnlichen Luftbefeuchter für Beatmungsgeräte, wie er in der DE 199 36 499 A1 beschrieben ist. Auch beim Luftbefeuchter aus der DE 200 10 553 U1 wird Luft über die Oberfläche eines beheizbaren Wasserreservoirs geführt. Anstelle der Nachfülleinheit, die aus einem Wannenelement und einem Topfteil besteht, wird ein Wasserbehälter verwendet, der im Wesentlichen aus einem Teil besteht.

Weitere ähnliche Luftbefeuchter sind aus der DE 101 51 397 C1 und dem deutschen Gebrauchsmuster 20 2004 004 115.4 bekannt.

Es ist Aufgabe der Erfindung einen Verdunster sowie ein Verdunstungsverfahren anzugeben, bei dem schnell Betriebsbereitschaft erreicht wird.

Diese Aufgabe wird durch die Lehre der unabhängigen Ansprüche gelöst.

Bevorzugte Ausführungsformen der Erfindung sind Gegenstand der Unteransprüche.

Auf Grund der geringen Wärmekapazität des zugeführten Gases verglichen mit der Wärmekapazität des Flüssigkeitsvorrats wird praktisch sofort nach Inbetriebnahme eine nennenswerte Verdunstung erreicht.

Durch die Beheizung des eintretenden Gases wird die gesamte Flüssigkeitsoberfläche einschließlich des Eintrittsbereichs für die Verdunstung von Flüssigkeitsmolekülen oder -atomen genutzt. Wird andererseits - wie bei herkömmlichen Luftbefeuchtern vorgesehen - die Flüssigkeit beheizt, wird das Gas im Eintrittbereich auf die Temperatur der Flüssigkeit erwärmt, sodass der Eintrittsbereich nicht effektiv für die Verdunstung genutzt wird, weil kälteres Gas weniger bereitwillig Flüssigkeitsmoleküle oder -atome aufnimmt.

Eine herausnehmbare innere Wanne, die als Flüssigkeitsvorratsbehälter dient, erleichtert das Nachfüllen von Flüssigkeit und die Reinigung des Verdunsters.

Druckdichtigkeit sorgt dafür, dass sich ein Verdunster für das Gas wie ein Stück Rohr verhält.

Durch eine gleichmäßige Heizung der äußeren Wanne wird die erforderliche Heizleistung durch eine große Fläche geleitet. Dies führt zu einer geringeren Temperatur an dem Heizer.

Vorteilhaft für eine billige Herstellung ist, dass zwischen der inneren Wanne und dem Deckel keine Dichtung erforderlich ist, sondern sogar ein schmaler Spalt frei bleiben kann. Vielmehr ist der unterschiedliche Gaswiderstand zwischen dem schmalen Spalt und dem Durchlass ausreichend dafür, dass der überwiegende Teil des Gases durch den Durchlass strömt.

Ferner ist bei den erfindungsgemäßen Ausführungsformen weder beim Ein- noch beim Auslass eine bewegte Dichtung erforderlich, was Herstellungskosten gering hält und die Zuverlässigkeit erhöht.

Der Wärmetransport von der beheizten äußeren Wanne zur inneren Wanne mit der Flüssigkeit wird dadurch unterstützt, dass sich das Gas zwischen äußerer und innerer Wanne bewegt, und zwar möglichst im gesamten Spalt. Durch lateralen Materialtransport in einer turbulenten Gasströmung ist die Wärmeleitfähigkeit von bewegten Gasen höher als von ruhenden Gasen. Zusätzlich wird Wärme durch den Gasfluss durch den Durchlass transportiert und vom Boden der äußeren Wanne auf den Boden der inneren Wanne übertragen. Dies alles sorgt für eine gute thermische Kopplung zwischen Heizer, Gas und Flüssigkeit, wodurch geringere Heizertemperaturen ausreichen, um eine Verdunstungskälte zu kompensieren. Geringe Heizertemperaturen reduzieren auch Probleme mit der Verkalkung.

Durch eine Anordnung des Durchlasses in Umlaufrichtung des Gases gesehen vor dem Einlass wird erreicht, das sich das Gas über die gesamte Spaltlänge hinweg bewegt.

Eine hohe Relativgeschwindigkeit zwischen Flüssigkeit und Gas begünstigt eine Verdunstung von Flüssigkeitsmolekülen oder -atomen.

Eine innere Wanne mit zwei zylinderförmigen Bereichen und einem dazwischen liegenden taillenförmigen Bereich passt gut in eine ovale äußeren Wanne. Der Spalt zwischen innerer und äußerer Wanne kann in den zylinderförmigen Bereichen schmal sein, was auf Grund des geringeren Abstands zwischen innerer und äußeren Wanne sowie der hohen Strömungsgeschwindigkeit des Gases und der daraus resultierenden Turbulenzen für einen guten Wärmeübergang sorgt. Im taillenförmigen Bereich ist der Abstand zwischen innerer und äußerer Wanne größer, sodass hier der Spalt breit genug für einen Normfinger ist, sodass die innere Wanne leicht zum Reinigen oder Nachfüllen von Wasser herausgenommen werden kann.

Die beiden Wannen und der Deckel können vorteilhafterweise aus Edelstahl hergestellt sein. Edelstahl hält verglichen mit Kunststoffen hohe Temperaturen aus und ist zusätzlich biokompatibel. Insbesondere Brandschutzzusätze für Kunststoffe sind meist nicht biokompatibel.

Im Folgenden werden bevorzugte Ausführungsformen der Erfindung unter Bezugnahme auf die beiliegenden Zeichnungen näher erläutert. Dabei zeigen:
Fig. 1 eine Draufsicht auf einen erfindungsgemäßen Verdunster;
Fig. 2 eine Seitenansicht des in Fig. 1 dargestellten Verdunsters;
Fig. 3 eine Draufsicht auf einen weiteren erfindungsgemäßen Verdunster;
Fig. 4 eine Seitenansicht des in Fig. 3 dargestellten Verdunsters; und
Figuren 5 bis 7 vertikale Schnitte durch verschiedene erfindungsgemäße Ausführungsformen der inneren Wanne.

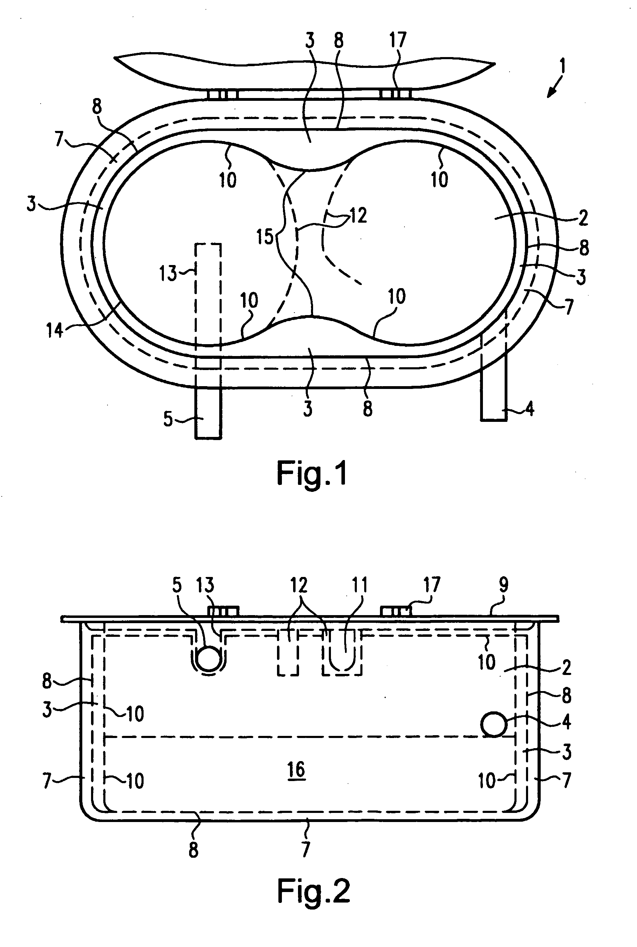

Fig. 1 und 2 zeigen eine Ausführungsform eines erfindungsgemäßen Verdunsters. Das Gehäuse 1 des Verdunsters besteht im wesentlichen aus drei Teilen, nämlich einer äußeren Wanne 8, einer inneren Wanne 10 sowie einem Deckel 9. Die innere Wanne 10 steht in der äußeren Wanne 8, so dass sich die beiden Wannenböden berühren und auch über die Böden Wärme übertragen werden kann. in Figur 1 ist Deckel 9 nur teilweise und zwar in aufgeklappter Stellung dargestellt. Deckel 9 kann mit Scharnieren 17 an äußerer Wanne 8 befestigt sein.

Zwischen den Mantelflächen der beiden Wannen bildet sich ein Spalt, der als Heizraum 3 bezeichnet wird. Der von der inneren Wanne 10 und dem Deckel 9 umschlossene Raum wird als Verdunstungsraum 2 bezeichnet. Im unteren Teil des Verdunstungsraums befindet sich eine Flüssigkeit, zumeist Wasser 16. Außen an der äußeren Wanne 8, also sowohl an der Boden- als auch der Mantelfläche ist ein Heizer 7 angebracht.

Gas, zumeist Luft, wird dem Verdunster über Einlass 4 zugeführt. Das Gas gelangt zunächst in Heizraum 3, wo es fast einmal gegen den Uhrzeigersinn um innere Wanne 10 herum fließt. Hierbei erwärmt sich das Gas an der Mantelfläche der äußeren Wanne 8 und überträgt einem Teil der Wärme auch an die innere Wanne 10. Anschließend fließt das Gas durch Durchlass 11 vom Heizraum 3 in den Verdunstungsraum 2. Durch Einbauten 12 im Deckel 9, deren Position bei geschlossenem Deckel 9 gestrichelt in Figur 1 eingetragen ist, wird erreicht, dass das Gas möglichst über die gesamte Flüssigkeitsoberfläche geführt wird. Dies begünstigt die Verdunstung der Flüssigkeit. Schließlich wird das Gas über Auslasskanal 13 und Auslass 5 abgeführt.

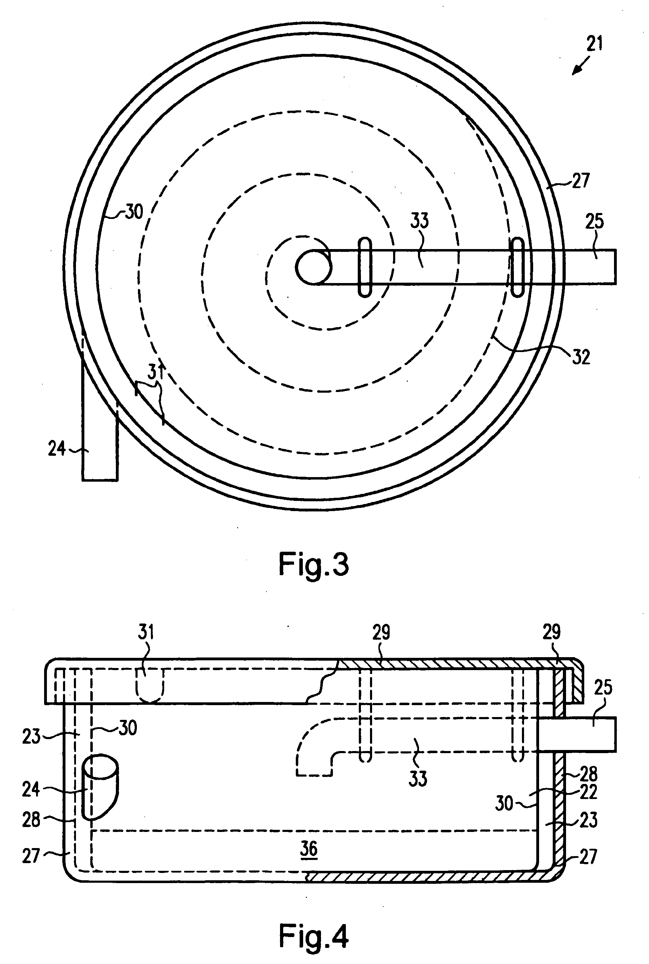

Eine andere Ausführungsform kann spiegelsymmetrisch zu der in Figuren 1 und 2 dargestellten Ausführungsform aufgebaut sein, so dass das Gas im Uhrzeigersinn um innere Wanne 10 herum fließt, wie beispielsweise bei der in Figuren 3 und 4 dargestellten Ausführungsform.

Die einzige hochwertige und bewegte Dichtung befindet sich zwischen äußerer Wanne 8 und Deckel 9. Zwar soll der in Fig. 2 sichtbare Spalt zwischen innerer Wanne 10 und Deckel 9 möglichst schmal sein, damit das Gas definiert durch Durchlass 11 strömt. Andererseits beeinträchtigt es die Funktionen des Verdunsters nicht wesentlich, wenn beispielsweise die Hälfte des Gases durch den Spalt und die andere Hälfte durch Durchlass 11 strömt.

Wie in Figur 1 gut zu sehen ist, weist die innere Wanne 10 einen knochenförmigen Grundriss mit zwei zylinderförmigen Bereichen 14 und einem dazwischen liegenden taillenförmigen Bereich 15 auf. Zweck des taillenförmigen Bereichs 15 ist vor allem, dass Normfinger im taillenförmigen Bereich 15 zwischen innerer und äußerer Wanne Platz haben. Dies wiederum stellt sicher, dass die innere Wanne bequem zum Nachfüllen oder zum Reinigen aus der äußeren Wanne herausgenommen werden kann.

Der taillenförmige Bereich 15 sorgt auch dafür, dass die Gasströmung in Heizraum 3 turbulenter wird. Deshalb wird die Wärmeübertragung von der Mantelfläche der äußeren Wanne über das Gas in Heizraum 3 auf die Mantelfläche der inneren Wanne nicht in dem Maße schlechter, wie es der größere Abstand zwischen den beiden Mantelflächen im taillenförmigen Bereich vermuten ließe.

Damit das Gas möglichst die gesamte Flüssigkeitsoberfläche überstreicht, befindet sich Durchlass 11 in dem einen und der Auslasskanal 13 in dem anderen zylinderförmigen Bereich.

Figuren 3 und 4 zeigen eine andere Ausführungsform des erfindungsgemäßen Verdunsters. Im Unterschied zu dem in Figuren 1 und 2 dargestellten Verdunster sind bei diesem Verdunster das Gehäuse 21 mit äußerer Wanne 28, innerer Wanne 30 sowie Deckel 29 im wesentlichen rotationssymmetrisch. Auch hier wird Gas über einen Einlass 24 in einen ringförmigen Heizraum 23 zwischen den Mantelflächen der inneren und äußeren Wanne geblasen. Nachdem das Gas fast einmal um die innere Wanne 30 herum geflossen ist, tritt es durch Durchlass 31 in Verdunstungsraum 22 innerhalb der inneren Wanne 30 und streicht dort über die Oberfläche einer Flüssigkeit, beispielsweise Wasser 36. Anschließend wird das befeuchtete oder mit Flüssigkeitsmolekülen oder -atomen angereicherte Gas aus dem Zentrum der beiden Wannen durch Auslasskanal 33 zu Auslass 25 geleitet. Auch diese Ausführungsform kann gespiegelt werden.

Deckel 29 kann spiralförmige Einbauten 32 aufweisen, die in Fig. 3 gestrichelt dargestellt sind, um die gesamte Flüssigkeitsoberfläche zur Verdunstung zu nutzen.

Wie in den Figuren 1 bis 4 dargestellt, werden insbesondere bei den Wannen, aber auch bei den Deckeln scharfe Ecken vermieden, um das Reinigen der Teile zu erleichtern. Insbesondere sind die Kanten zwischen den Mantelflächen und den Böden der Wannen verrundet. Diese Reinigungsfreundlichkeit ermöglicht es, den Verdunster fest in ein größeres Gerät einzubauen und damit bewegte Dichtungen bei Einlass und Auslass zu vermeiden.

Die äußeren und inneren Wannen sowie der Deckel können aus Edelstahl hergestellt werden. Edelstahl ist biokompatibel und verträgt hohe Temperaturen. Brandschutzstoffe in Kunststoffen sind dagegen meist nicht biokompatibel.

Heizer 7 kann beispielsweise aus gewickeltem Heizdraht, der mit Klebeband fixiert und isoliert wird oder aus einer Kapton-Folienheizung bestehen.

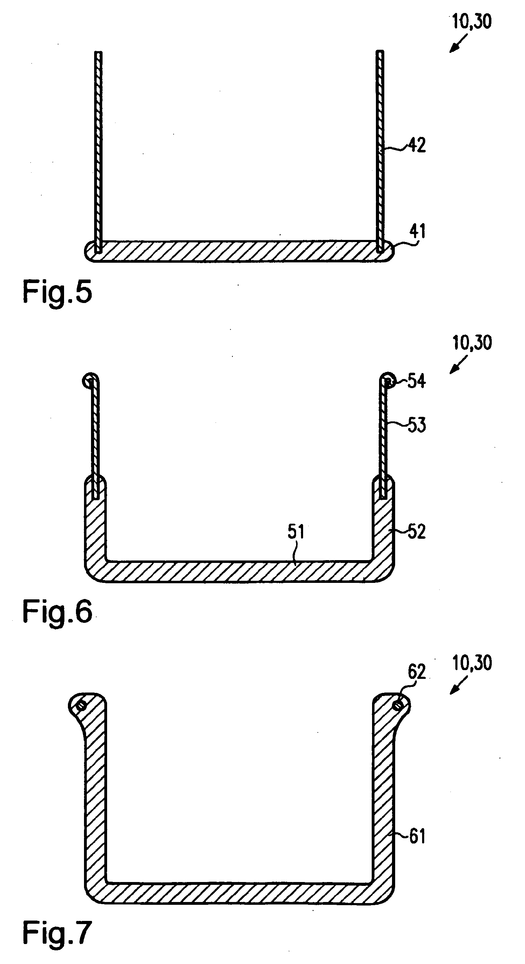

Die in Figuren 1 bis 4 nur schematisch dargestellte innere Wanne kann auch wie in einer der Figuren 5 bis 7 dargestellt ausgeführt sein. Figuren 5 bis 7 zeigen vertikale Schnitte durch innere Wannen 10 oder 30.

Bei den in Figuren 5 bis 7 dargestellten Ausführungsformen ist der Boden der inneren Wanne aus einem elastischen, biokompatiblen Material, beispielsweise Silicon hergestellt. Dies hat den Vorteil, dass sich der Boden der inneren Wanne dem Boden der äußeren Wanne anpasst und ein Luftspalt zwischen innerer und äußerer Wanne verkleinert und im Idealfall über die ganze Bodenfläche auf Null reduziert wird. Hierdurch wird der Wärmewiderstand zwischen den beiden Böden reduziert und die Heizertemperatur weiter abgesenkt.

Obwohl der Boden der inneren Wanne weich sein soll, um sich dem Boden der äußeren Wanne anzupassen, müssen die Wände der inneren Wanne eine gewisse Steifigkeit aufweisen, damit die innere Wanne beim Herausnehmen aus der äußeren Wanne gegriffen werden kann.

Auch der Boden der äußeren Wanne kann aus einem weichen, elastischen Material wie Silicon hergestellt oder mit einem solchen Material beschichtet sein. Eine solche äußerer Wanne kann mit einer inneren Wanne aus Edelstahl oder mit einer inneren Wanne, deren Boden aus Silikon besteht, kombiniert werden.

Fig. 5 zeigt eine Ausführungsform der inneren Wanne, bei der der Boden 41 aus Silicon, die Wände 42 jedoch aus Edelstahl gefertigt sind.

Fig. 6 zeigt eine Ausführungsform der inneren Wanne, bei der der Boden 51 sowie der untere Teil der Mantelfläche 52 aus Silicon, der obere Teil der Mantelfläche 53 jedoch aus Edelstahl gefertigt ist. Der obere Rand 54 der Mantelfläche kann umgebogen sein.

Bei der in Fig. 7 dargestellten Ausführungsform besteht die gesamte innere Wanne aus Silicon 61. Der obere Rand der Wanne ist verbreitert, um hier die Stabilität zu erhöhen. Um die Stabilität weiter zu erhöhen, kann in den oberen Rand ein Draht 62, insbesondere ein Federdraht eingelassen sein.

Auf Grund des Heizens des Gases wird praktisch unmittelbar nach Inbetriebnahme eine nennenswerte Verdunstung der Flüssigkeit erreicht. Nach Inbetriebnahme steigt die Verdunstungsrate an, bis die Flüssigkeit ihre Gleichgewichtstemperatur erreicht hat. Um diesen Prozess zu beschleunigen, kann es vorteilhaft sein, vor Inbetriebnahme drei bis vier Minuten vorzuheizen.

Zwar werden die erfindungsgemäßen Verdunster hauptsächlich als Luftbefeuchter eingesetzt. Dies ist jedoch kein notwendiges Merkmal, da aus der deutschen Patentanmeldung 103 22 964.7 bekannt ist, ein Gasgemisch mit einer von Luft abweichenden Zusammensetzung zu verwenden oder der Luft Wirkstoffe wie Aerosole, Duftstoffe oder Medizin zuzusetzen. Auch dem Wasser kann ein Wirkstoff wie beispielsweise Meerwasserzusatz zugegeben sein, sodass der Oberbegriff "Flüssigkeit" gerechtfertigt ist.

Die Erfindung wurde zuvor anhand von bevorzugten Ausführungsformen näher erläutert. Für einen Fachmann ist jedoch offensichtlich, dass verschiedene Abwandlungen und Modifikationen gemacht werden können, ohne vom Geist der Erfindung abzuweichen. Deshalb wird der Schutzbereich durch die nachfolgenden Ansprüche und ihre Äquivalente festgelegt.

### Bezugszeichenliste

- 1: Gehäuse
- 2: Verdunstungsraum
- 3: Heizraum
- 4: Einlass
- 5: Auslass
- 7: Heizer
- 8: äußere Wanne
- 9: Deckel
- 10: innere Wanne
- 11: Durchlass
- 12: Einbauten
- 13: Auslasskanal
- 14: zylinderförmige Bereiche
- 15: taillenförmiger Bereich
- 16: Wasser
- 17: Scharnier
- 21: Gehäuse
- 22: Verdunstungsraum
- 23: Heizraum
- 24: Einlass
- 25: Auslass
- 27: Heizer
- 28: äußere Wanne
- 29: Deckel
- 30: innere Wanne
- 31: Durchlass
- 32: Einbauten
- 33: Auslasskanal
- 36: Wasser
- 41: Boden
- 42: Mantelfläche
- 51: Boden
- 52: untere Mantelfläche
- 53: oberen Mantelfläche
- 54: umgebogener Rand
- 61: Silicon
- 61: Draht

## Patentansprüche

1. Verdunster mit:
einem Heizer (7; 27);
einem Gehäuse (1; 21) mit einem Einlass (4; 24) zum Zuführen eines Gases sowie einem Auslass (5; 25) zum Abführen des zugeführten Gases; wobei das Gehäuse (1; 21) einen Verdunstungsraum (2; 22) bildet, der teilweise mit einer Flüssigkeit (16; 36) gefüllt werden kann; wobei das Gehäuse (1; 21) einen Heizraum (3; 23) bildet, der in thermischem Kontakt mit dem Heizer (7; 27) steht, wobei der Einlass (4; 24) mit dem Heizraum (3; 23), und der Verdunstungsraum (2; 22) mit dem Auslass (5; 25) pneumatisch verbunden ist, dass Gas vom Einlass (4; 24) über den Heizraum (3; 23) zum Verdunstungsraum (2; 22) und weiter zum Auslass (5; 25) strömen kann; **dadurch gekennzeichnet, dass** das Gehäuse (1; 21) eine oben offene und unten dichte innere Wanne (10; 30) zur Aufnahme der Flüssigkeit (16; 36) umfasst, wobei die Mantelfläche der inneren Wanne (10; 30) den Verdunstungsraum (2; 22) vom Heizraum (3; 23) abgrenzt.

2. Verdunster gemäß Anspruch 1, **dadurch gekennzeichnet, dass** das Gehäuse (1; 21) ferner eine äußere Wanne (8; 28) und einen Deckel (9; 29) umfasst, wobei die äußere Wanne (8; 28) und der Deckel (9; 29) druckdicht schließen.

3. Verdunster gemäß Anspruch 2, **dadurch gekennzeichnet, dass** der Heizer (7; 27) außen an der äußeren Wanne (8; 28) angebracht ist, sodass der Heizer (7; 27) die äußere Wanne (8; 28) weitgehend gleichmäßig heizt.

4. Verdunster gemäß Anspruch 3, **dadurch gekennzeichnet, dass** der Einlass (4; 24) so geformt ist, dass in den Heizraum (3; 23) eintretendes Gas tangential, also in etwa parallel zu den Wänden der inneren und äußeren Wanne (10, 8; 30, 28), in den Heizraum eintritt, so dass durch die formgebung des Einlasses (4; 24) eine Umlaufrichtung des Gases im Heizraum um die innere Wanne (10; 30) festgelegt ist.

5. Verdunster gemäß Anspruch 4, **dadurch gekennzeichnet, dass** sich ein Durchlass (11; 31) in der inneren Wanne (10; 30) befindet, wobei der Durchlass (11; 31) den Heizraum (3; 23) mit dem Verdunstungsraum (2; 22) verbindet und wobei sich der Durchlass in Umlaufrichtung gesehen kurz vor dem Einlass (4; 24) befindet.

6. Verdunster gemäß einem der Ansprüche 2 bis 5, **dadurch gekennzeichnet, dass** der Deckel (9; 29) Einbauten (12; 32) zur Gasleitung aufweist, die im Betrieb für eine hohe Relativgeschwindigkeit zwischen der Flüssigkeit (16; 36) und dem zugeführten Gas sorgen.

7. Verdunster gemäß Anspruch 5 oder Ansprüchen 5 und 6, **dadurch gekennzeichnet, dass** die Mantelfläche der inneren Wanne (10) zwei zylinderförmige Bereiche (14) aufweist, die durch einen taillenförmigen Bereich (15) verbunden sind, wobei sich der Durchlass (11) in dem einen zylinderförmigen Bereich befindet und sich ein Auslasskanal (13) vom Zentrum des anderen zylinderförmigen Bereichs zum Auslass (5) erstreckt.

8. Verdunster gemäß Anspruch 5 oder Ansprüchen 5 und 6, **dadurch gekennzeichnet, dass** die Mantelflächen der inneren und äußeren Wanne (10, 8) zylinderförmig sind und sich ein Auslasskanal (33) vom Zentrum der inneren Wanne (30) zum Auslass (25) erstreckt.

9. Verdunster gemäß einem der obigen Ansprüche, **dadurch gekennzeichnet, dass** die innere Wanne (10; 30), die äußere Wanne (8; 28) und/oder der Deckel (9; 29) aus Edelstahl gefertigt sind.

10. Verdunster gemäß einem der obigen Ansprüche, **dadurch gekennzeichnet, dass** die äußere Wanne (8; 28) aus Edelstahl und die Bodenfläche (41; 51; 61) der inneren Wanne (10; 30) aus Silicon gefertigt ist.

11. Verdunstungsverfahren mit:
Heizen (7; 27) eines Gases in einem Heizraum (3; 33),
Führen (2; 22) des Gases über eine Flüssigkeitsoberfläche, nachdem es im Heizraum (3; 33) geheizt wurde;
**dadurch gekennzeichnet dass**
sich der Heizraum (3; 33) zwischen einer inneren Wanne (10; 30) und einer äußeren Wanne (8; 28) befindet;
die äußere Wanne (8; 28) beheizt wird; und
das Gas in einem Verdunstungsraum (2; 22) über die Flüssigkeitsoberfläche geführt wird; wobei sich der Verdunstungsraum und die Flüssigkeit (16; 36) innerhalb der inneren Wanne (10; 30) befinden.

## Claims

1. An evaporator, comprising
a heater (7; 27);
a casing (1; 21) having an inlet (4; 24) for supplying a gas and an outlet (5; 25) for discharging the supplied gas; wherein the casing (1; 21) forms an evaporating space (2; 22) which may partially be filled with a liquid (16; 36); wherein the casing (1; 21) forms a heating space (3; 23) being in thermal contact with the heater (7; 27), wherein the inlet (4; 24) is pneumatically connected to the heating space (3; 23) and the evaporating space (2; 22) is pneumatically connected to the outlet (5; 25) to allow gas to flow from the inlet (4; 24) through the heating space (3; 23) to the evaporating space (2; 22) and further to the outlet (5; 25);
**characterized in that**
the casing (1; 21) comprises an inner tub (10; 30) being open at the top and sealed at the bottom to receive the liquid (16; 36), wherein the circumferential surface of the inner tub (10; 30) delimits the evaporating space (2; 22) from the heating space (3; 23).

2. The evaporator according to claim 1, **characterized in that** the casing (1; 21) further comprises an outer tub (8; 28) and a lid (9; 29), wherein the outer tub (8; 28) and the lid (9; 29) are closable in a pressure-tight manner.

3. The evaporator according to claim 2, **characterized in that** the heater (7; 27) is attached outside on the outer tub (8; 28) so that the heater (7; 27) heats the outer tub (8; 28) largely uniformly.

4. The evaporator according to claim 3, **characterized in that** the inlet (4; 24) is formed such that gas flowing into the heating space (3; 23) flows into the same tangentially, i.e. approximately parallel to the walls of the inner and the outer tub (10, 8; 30, 28) so that the design of the inlet (4; 24) defines a direction of circulation of the gas in the heating space around the inner tub (10; 30).

5. The evaporator according to claim 4, **characterized in that** an aperture (11; 31) is located in the inner tub (10; 30), wherein the aperture (11; 31) connects the heating space (3; 23) to the evaporating space (2; 22) and wherein the aperture is located just backward of the inlet (4; 24) seen in the direction of circulation.

6. The evaporator according to one of claims 2 to 5, **characterized in that** the lid (9; 29) is provided with internal parts (12; 32) for conducting the gas, which provide for a high relative speed between the liquid (16; 36) and the supplied gas during operation.

7. The evaporator according to claim 5 or claims 5 and 6, **characterized in that** the circumferential surface of the inner tub (10) has two cylindrical portions (14) connected by a waisted portion (15), wherein the aperture (11) is located in the one cylindrical portion and an outlet channel (13) extends from the center of the other cylindrical portion toward the outlet (5).

8. The evaporator according to claim 5 or claims 5 and 6, **characterized in that** the circumferential surfaces of the inner and outer tub (10, 8) are cylindrical and an outlet channel (33) extends from the center of the inner tub (30) to the outlet (25).

9. The evaporator according to one of the preceding claims, **characterized in that** the inner tub (10; 30), the outer tub (8; 28) and/or the lid (9; 29) are made of stainless steel.

10. The evaporator according to one of the preceding claims, **characterized in that** the outer tub (8; 28) is made of stainless steel and the bottom surface (41; 51; 61) of the inner tub (10; 30) is made of silicone.

11. An evaporation method, comprising
heating (7; 27) a gas in a heating space (3; 33),
conducting (2; 22) the gas over a liquid surface after it was heated in the heating space (3; 33);
**characterized in that**
the heating space (3; 33) is located between an inner tub (10; 30) and an outer tub (8; 28);
the outer tub (8; 28) is heated; and
the gas is conducted in an evaporating space (2; 22) over the liquid surface; wherein the evaporating space and the liquid (16; 36) are located inside the inner tub (10; 30).

## Revendications

1. Évaporateur avec :
un dispositif de chauffe (7; 27) ;
un boîtier (1 ; 21) doté d'une admission (4 ; 24) pour amener un gaz ainsi qu'une évacuation (5 ; 25) pour évacuer le gaz amené ; le boîtier (1 ; 21) formant une chambre d'évaporation (2 ; 22) pouvant en partie être remplie avec un liquide (16 ; 36) ; le boîtier (1 ; 21) formant une chambre de chauffe (3 ; 23) en contact thermique avec le dispositif de chauffe (7 ; 27), l'admission (4 ; 24) étant reliée de façon pneumatique à la chambre de chauffe (3 ; 23), et la chambre d'évaporation (2 ; 22) à l'évacuation (5 ; 25), le gaz pouvant circuler de l'admission (4 ; 24) à la chambre d'évaporation (2 ; 22) puis à l'évacuation (5 ; 25) en passant par la chambre de chauffe (3 ; 23) ;
**caractérisé en ce que** le boîtier (1 ; 21) comprend un intérieur de cuve (10 ; 30) ouvert vers le haut et étanche vers le bas pour recevoir le liquide (16 ; 36), la surface de revêtement de l'intérieur de cuve (10 ; 30) délimitant la chambre d'évaporation (2 ; 22) et la chambre de chauffe (3 ; 23).

2. Évaporateur selon la revendication 1, **caractérisé en ce que** le boîtier (1 ; 21) comprend en outre un extérieur de cuve (8 ; 28) et un cache (9 ; 29), l'extérieur de cuve (8 ; 28) et le cache (9 ; 29) fermant par établissement d'une pression étanche.

3. Évaporateur selon la revendication 2, **caractérisé en ce que** le dispositif de chauffe (7 ; 27) extérieur est amené au niveau de l'extérieur de cuve (8 ; 28) de sorte que le dispositif de chauffe (7 ; 27) chauffe de façon largement régulière l'extérieur de cuve (8 ; 28).

4. Évaporateur selon la revendication 3, **caractérisé en ce que** l'admission (4 ; 24) est formée de telle sorte que le gaz entrant dans la chambre de chauffe (3 ; 23) entre tangentiellement, donc approximativement parallèlement aux parois de l'intérieur et l'extérieur de cuve (10, 8 ; 30, 28), dans la chambre de chauffe, de sorte que la formation de l'admission (4 ; 24) permet de fixer une direction de circulation circonférentielle du gaz dans la chambre de chauffe autour de l'intérieur de cuve (10 ; 30).

5. Évaporateur selon la revendication 4, **caractérisé en ce qu'**un passage (11 ; 31) se trouve dans l'intérieur de cuve (10 ; 30), le passage (11 ; 31) reliant la chambre de chauffe (3 ; 23) à la chambre d'évaporation (2 ; 22) et le passage se trouvant, vu dans la direction de circulation circonférentielle, juste avant l'admission (4 ; 24).

6. Évaporateur selon l'une quelconque des revendications 2 à 5, **caractérisé en ce que** le cache (9 ; 29) comprend des encastrements (12 ; 32) pour conduire le gaz, lesdits encastrements permettant d'atteindre en fonctionnement une vitesse relativement élevée entre le liquide (16 ; 36) et le gaz amené.

7. Évaporateur selon la revendication 5 ou les revendications 5 et 6, **caractérisé en ce que** la surface de revêtement de l'intérieur de cuve (10) comporte deux zones (14) cylindriques reliées par une zone (15) en forme de taille, le passage (11) se trouvant dans une zone cylindrique et un canal d'évacuation (13) s'élargissant du centre de l'autre zone cylindrique vers l'évacuation (5).

8. Évaporateur selon la revendication 5 ou les revendications 5 et 6, **caractérisé en ce que** les surfaces de revêtement de l'intérieur et l'extérieur de cuve (10, 8) prennent une forme cylindrique et qu'un canal d'évacuation (33) s'élargit du centre de l'intérieur de cuve (30) vers l'évacuation (25).

9. Évaporateur selon l'une quelconque des revendications précédentes, **caractérisé en ce que** l'intérieur de cuve (10 ; 30), l'extérieur de cuve (8 ; 28) et/ou le cache (9 ; 29) sont fabriqués en acier inoxydable.

10. Évaporateur selon l'une quelconque des revendications précédentes, **caractérisé en ce que** l'extérieur de cuve (8 ; 28) est en acier inoxydable et que la surface de fond (41 ; 51 ; 61) de l'intérieur de cuve (10 ; 30) est fabriquée en silicone.

11. Procédé d'évaporation comprenant les étapes suivantes :
chauffage (7 ; 27) d'un gaz dans une chambre de chauffe (3 ; 33), amenée (2 ; 22) du gaz sur une surface liquide après son chauffage dans la chambre de chauffe (3 ; 33) ;
**caractérisé en ce que** :
la chambre de chauffe (3 ; 33) se situe entre un intérieur de cuve (10 ; 30) et un extérieur de cuve (8 ; 28) ;
l'extérieur de cuve (8 ; 28) est chauffé ; et
le gaz est amené dans une chambre d'évaporation (2 ; 22) en passant par la surface de liquide ; la chambre d'évaporation et le liquide (16 ; 36) se trouvant dans l'intérieur de cuve (10 ; 30).
